# EUROPÄISCHE PATENTSCHRIFT

(11) **EP 1 131 307 B1**
(45) Veröffentlichungstag und Bekanntmachung des Hinweises auf die Patenterteilung: **25.08.2004**
(21) Anmeldenummer: 99960983.7
(22) Anmeldetag: 17.11.1999
(51) Int. Cl.: C07D 261/04

(54) **VERFAHREN ZUR HERSTELLUNG VON 2-ALKYL- 3-(4,5- DIHYDROISOXAZOL- 3-YL)-HALOGENBENZOLEN**
METHOD OF PRODUCING 2-ALKYL-3-(4,5-DIHYDROISOXAZOLE-3-YL)-HALOBENZENES
PROCEDE DE PRODUCTION DE 2-ALKYL-3-(4,5-DIHYDROISOXAZOL-3-YL)-HALOGENOBENZENES

(30) Priorität: 18.11.1998 DE 19853039
(43) Veröffentlichungstag der Anmeldung: 12.09.2001
(73) Patentinhaber: BASF AKTIENGESELLSCHAFT, 67056 Ludwigshafen (DE)
(72) Erfinder: RACK, Michael, D-69123 Heidelberg (DE); GÖTZ, Norbert, D-67547 Worms (DE); HAGEN, Helmut, D-67227 Frankenthal (DE); VON DEYN, Wolfgang, D-67435 Neustadt (DE); BAUMANN, Ernst, D-67373 Dudenhofen (DE); LOCHTMAN, Rene, D-68161 Mannheim (DE); GEBHARDT, Joachim, D-67157 Wachenheim (DE)
(86) Internationale Anmeldenummer: PCT/EP1999/008844
(87) Internationale Veröffentlichungsnummer: WO 2000/029395

(56) Entgegenhaltungen:
- WO-A-96/26200
- WO-A-98/31676
- WO-A-99/58509

## Beschreibung

Gegenstand der vorliegenden Erfindung ist ein Verfahren zur Herstellung von 2-Alkyl-3-(4,5-dihydroisoxazol-3-yl)-halogenbenzolen.

2-Alkyl-3-(4,5-dihydroisoxazol-3-yl)-halogenbenzole sind Ausgangsprodukte zur Herstellung von 2-Alkyl-3-(4,5-dihydroisoxazol-3-yl)-acylbenzolen, die im Bereich des Pflanzenschutzes eingesetzt werden können. Derartige Verbindungen werden beispielsweise in WO 98/31681 als herbizide Wirkstoffe beschrieben.

Weiterhin ist aus WO 98/31676 die Darstellung von 2-Chlor-3-(4,5-dihydroisoxazol-3-yl)halogenbenzolen bekannt.

Aufgabe der vorliegenden Erfindung war es, ein verbessertes Herstellungsverfahren für 2-Alkyl-3-heterocyclyl-substituierte Benzoylderivate zur Verfügung zu stellen, wie sie beispielsweise in WO 98/31681 beschriebenen sind. Das in WO 98/31681 beschriebene Herstellungsverfahren der 2-Alkyl-3-(4,5-dihydroisoxazol-3-yl)-acylbenzolen bzw. deren Vorstufe (2-Alkyl-3-(4,5-dihydroisoxazol-3-yl)-brombenzolen) ist für die großtechnische Herstellung dieser Verbindungen nicht optimal, da die Synthese über mehrere Stufen erfolgt und die Ausbeute des jeweiligen Endproduktes, bezogen auf die in der ersten Synthesestufe eingesetzten Ausgangsprodukte, relativ niedrig ist.

Überraschenderweise wurde gefunden, daß nach dem erfindungsgemäßen Verfahren die 2-Alkyl-3-heterocyclyl-substituierten Benzoylderivate bzw. deren verschiedenen Vorstufen in guter Ausbeute und in einem vorteilhaften wirtschaftlichen Maßstab hergestellt werden können. Das erfindungsgemäße Verfahren hat den Vorteil, daß die Gesamtausbeute der jeweiligen Endprodukte, bezogen auf die eingesetzten Ausgangsstoffe, höher ist als die Ausbeute nach den in WO 98/31681 beschriebenen Verfahren. Ferner sind die Ausgangsstoffe einfach herstellbar bzw. können von mehreren unabhängigen Grundstofflieferanten auch in größeren Mengen bezogen werden, so daß insgesamt ein kostengünstigeres, wirtschaftliches und sicheres großtechnisches Verfahren zur Herstellung von herbiziden Wirkstoffen zur Verfügung steht.

Gegenstand der vorliegenden Erfindung ist ein Verfahren zur Herstellung der Verbindungen der Formel I 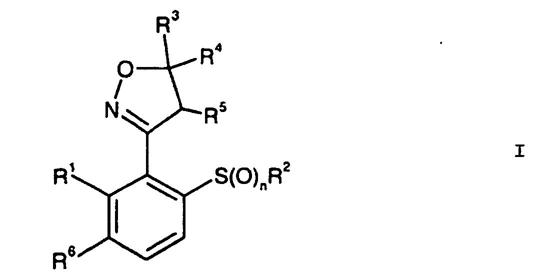 in der die Substituenten folgende Bedeutung haben:
- n: 0,1 oder 2;
- R¹,R²: C₁-C₆-Alkyl;
- R³,R⁴,R⁵: Wasserstoff, C₁-C₆-Alkyl, insbesondere Methyl, oder R⁴ und R⁵ bilden zusammen eine Bindung;
- R⁶: Cl, Br,
umfassend die aufeinander abfolgende Durchführung der Verfahrensschritte a) - c):
a) Halogenierung eines 1,2-Dialkylbenzols der Formel II 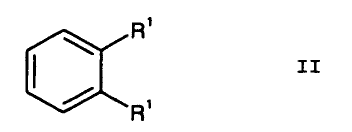 in der die Reste R¹ jeweils gleich oder verschieden sein können und die vorgegebene Bedeutung haben, mit Halogenen, insbesondere Chlor oder Brom, zu den 3,6-Dihalogen-1,2-dialkylbenzolen der Formel III 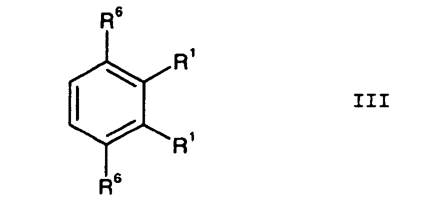
b) Umsetzung eines 3,6-Dihalogen-1,2-dialkylbenzol der Formel III mit Wasserstoffperoxid und einem Halogenierungsmittels, vorzugsweise HBr, zu den Benzylhalogeniden, insbesondere den Benzylbromiden, der Formel IV 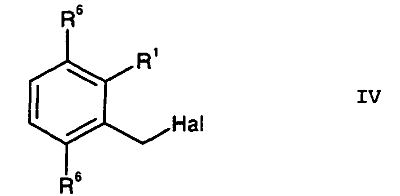 in denen die Reste R¹ und R⁶ die vorgenannte Bedeutung haben;
c) Oxidation der Benzylbromide der Formel IV mit einem Oxidationsmittel zu den Aldehyden der Formel V 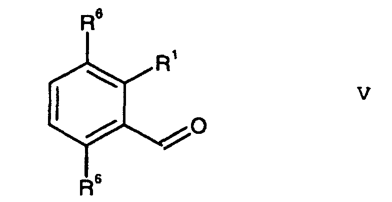 bei denen die Substiuenten R¹ und R⁶ die vorgenannte Bedeutung haben.
   In einer weiteren Ausgestaltungsform umfasst das oben genannte Verfahren zusätzlich einen oder mehrere der folgenden Verfahrensschritte:
d) Umsetzung der Verbindungen der Formel V mit Hydroxylamin und Base zu den entsprechenden Oximen der Formel VI 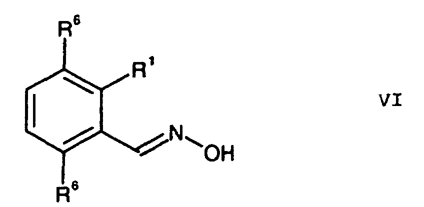 bei denen die Substituenten R¹ und R⁶ die vorgenannte Bedeutung haben;
e) Umsetzung der Oxime der Formel VI mit einem Alken der Formel VII 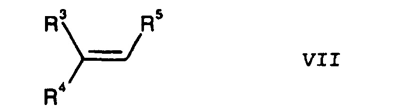 in der R³ bis R⁵ die oben genannten Bedeutungen haben, in Gegenwart eines Hypochlorits zu 4,5-Dihydroisoxazol der Formel VIII 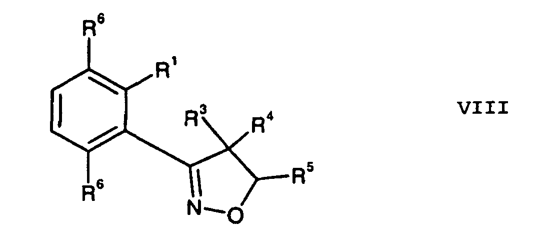 bei denen R¹ und R³ bis R⁶ die oben genannten Bedeutungen haben;
f) Umsetzung der Verbindung der Formel VIII mit Metallthiolaten der Formel IX

   R²―S⁻ M⁺ IX

   in Gegenwart eines Lösungsmittels zu den Thioethern der Formel X 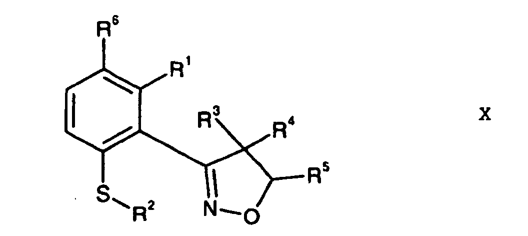 bei denen R¹ bis R⁶ die oben genannten Bedeutungen haben;
g) gegebenenfalls Umsetzung der Thioether der Formel X mit einem Oxidationsmittel zu den entsprechenden Alkylsulfonyl- oder Alkylsulfenylderivaten der Formel I, wobei n die Zahlen 1 oder 2 bedeutet.

C₁-C₆-Alkyl bedeutet in allen Fällen eine geradkettige oder verzweigte Alkylgruppen mit 1 - 6 C-Atomen, wie z.B. Methyl, Ethyl, n-Propyl, iso-Propyl, n-Butyl, iso-Butyl, n-Pentyl oder n-Hexyl. Analoges gilt für die C₁-C₆-Alkoxygruppe.

R⁴ und R⁵ können gemeinsam auch eine Bindung darstellen, so daß die entsprechenden Isoxazol-Derivate resultieren. In diesem Fall ist R³ bevorzugt Wasserstoff.

Die Reaktionsabfolge bis zu den Verbindungen der Formel I ist anhand des folgenden Übersichtsschemas zusammengestellt:

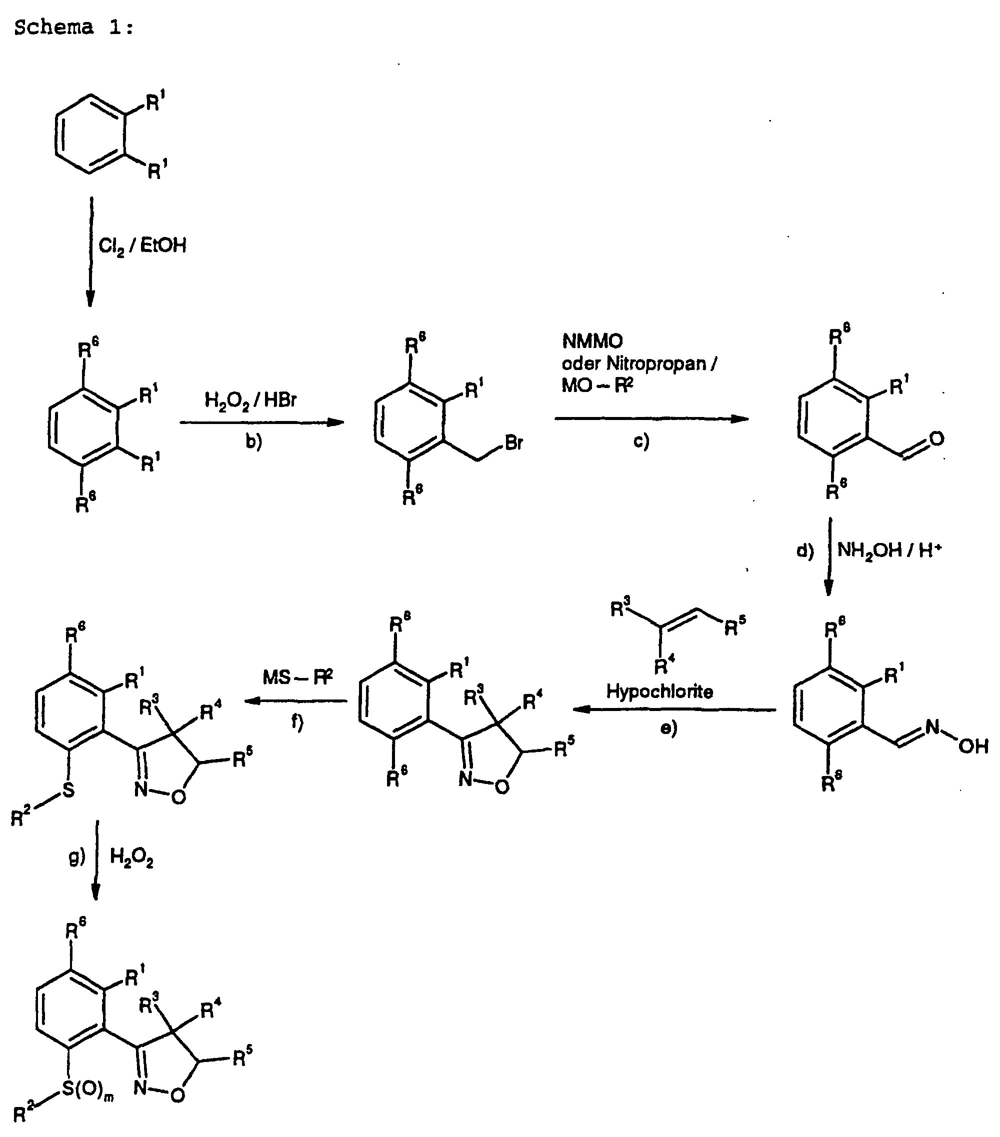

Im folgenden werden die einzelnen Reaktionsstufen kurz näher erläutert.
1. Stufe a)
   Die Halogenierung erfolgt nach literaturbekannten Methoden, vorzugsweise unter Verwendung von Chlorgas. Als Lösungsmittel eignen sich Alkohole, wie z.B. Ethanol.
2. Stufe b) 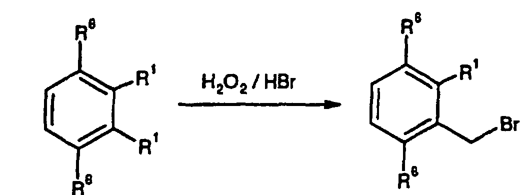
   Die Umsetzung erfolgt unter folgenden Bedingungen: Lösungsmittel: Bei der Bromierung inerte Lösungsmittel wie: Benzol, t.-Butylbenzol, t.-Amylbenzol, halogenierte Kohlenwasserstoffe, wie Methylenchlorid, Chloroform, Chlorbenzol 1,2-Dichlorethan, Tetrachlorkohlenstoff, Dichlorbenzol oder Trichlorbenzol. Auch Gemische dieser Lösungsmittel sind möglich. Bromierungsmittel: Brom, oder Bromsalze z.B. Bromsalze, HBr, bevorzugt in seiner wäßrigen Lösung. Besonders bevorzugt können auch technische azeotrope Mischungen von HBr eingesetzt werden.
3. Stufe c) 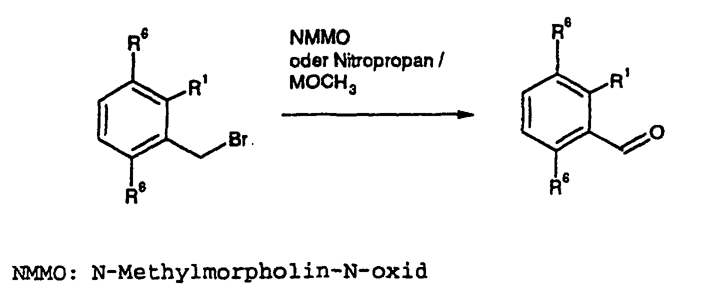
   Für die Oxidation sind z. B. Oxidationsmittel, wie Persäuren Peroxide, Hypochlorit, Chlor, Natriumbromat und Kaliumperoxodisulfat geeignet, besonders geeignet ist Wasserstoffperoxid. Aus der Literatur (DE-29 48 058) ist bekannt, daß man Alkylund Benzylhalogenide zu den entsprechenden Carbonylverbindungen mit Aminoxiden von tertiären Aminen oder Pyridin oxidieren kann. Die Umsetzung erfolgt unter folgenden Bedingungen: Aminoxide: Aminoxide mit aliphatischen, cycloaliphatischen und aromatischen Resten wie Trimethylamin, Dimethylcyclopentylamin, Dimethylamin. Weiterhin Aminoxide mit cycloaliphatischen Resten, die durch Heteroatome (O; N) unterbrochen sind. N-Alkyl und N-Arylsubstituierte Piperidine, Piperazine und Morpholine.
   Alternativ kann die in US 2,902,515 beschriebene Methode angewandt werden, nach der Allylhalogenide mit Alkalinitronaten zu den entsprechenden Aldehyden umgesetzt werden können. Die Bedingungen sind beispielsweise die folgenden: Lösungsmittel: Alkohole wie Methanol, Ethanol, Isopropanol, Ether wie Dioxan, THF, dipolar aprotische Lösungsmittel wie z.B. N,N-Dialkylformamide, -acetamide, N-Methylpyrrolidon, Dimethylpropylenharnstoff; Tetramethylharnstoff, DMF, NMP, Acetonitril. Bevorzugt Methanol. Die Erzeugung der Nitronate erfolgt wie folgt: Umsetzung von niederen Nitroalkanen mit Alkalimetallhydroxiden (wäßrige NaOH oder KOH) oder Umsetzung von niederen Nitroalkanen mit Alkalialkoholaten wie KotBu In Butanol oder Natriummethylat in Methanol. Die so erhaltenen Nitronate werden mit den Benzylhalogeniden umgesetzt. Die Umsetzung erfolgt bei Temperaturen zwischen -10? und 80°C bevorzugt bei 0°C bis 50?C. Die Aufarbeitung erfolgt wäßrig.
4. Stufe d) 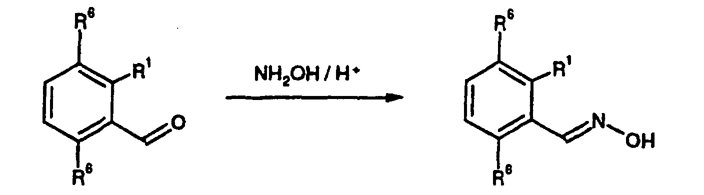
   Das Benzaldoxim der ist nach Standardverfahren ausgehend von den entsprechenden Aldehyden der durch Umsetzung mit Hydroxylamin in Gegenwart von Säure in nahezu quantitativer Ausbeute zugänglich.
5. Stufe e) 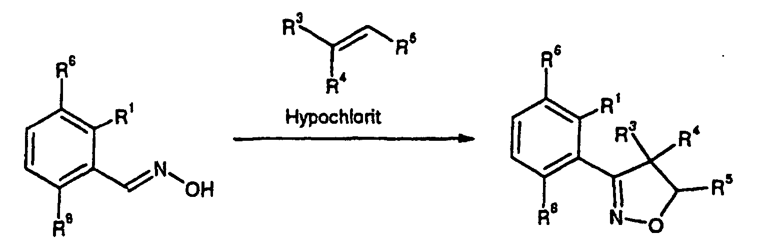
   Die Umsetzung des Benzaldoxims der Formel VI mit Alkenen der Formel VII zu Verbindungen der Formel VIII verläuft über verschiedene Zwischenstufen. Da der erste Reaktionsschritt die Bildung eines intermediären Hydroxamsäurehalogenides ist, müssen ein geeignetes oxidierendes Reagenz und eine Halogenquelle oder auch das Halogen selbst zugegen sein. Der zweite Reaktionsschritt ist dann die Eliminierung von Halogenwasserstoff zum Nitriloxid, die basische Bedingungen erfordert. Als Drittes folgt schließlich die Cycloaddition des Nitriloxids an das Alken.
   Nach üblichen Verfahren läßt sich diese sequenz schrittweise ausführen, wobei zur Bildung des Hydroxamsäurehalogenids z. B. die freien Halogene Brom oder Chlor eingesetzt werden können. Da die Hydroxamsäurehalogenide zur Zersetzung neigen, müssen sie dann bald mit einer Base weiter in die noch empfindlicheren Nitriloxide überführt werden, die man meist in situ mit dem Alken abfängt.
   Bei dem erfindungsgemäßen Verfahren wurden diese Einzelschritte nun vorteilhaft zu einer "Eintopfreaktion" zusammengefaßt. Dazu wird in der Regel in einem Lösungsmitteln wie beispielsweise Halogenalkanen wie Dichlorethan und Methylenchlorid oder Aromaten wie Benzol, Toluol, Chlorbenzol, Nitrobenzol oder Xylol gearbeitet, daß die organische Komponente löst, aber selbst nicht störend in die Reaktion eingreift. Als Halogenierungsmittel und gleichzeitig als Base wird eine wässrige Alkalihypohalogenid-Lösung, vorzugsweise 1-2 Äquivalente kommerziell verfügbare Natriumhypochlorid-Lösung, zugegeben, wobei das Alken parallel oder unmittelbar danach zugefügt wird. Das Reaktionsgemisch ist also üblicherweise zweiphasig, da sich das organische Lösungsmittel mit der Alkalihypohalogenid-Lösung nur unvollständig mischt. Zur Vervollständigung des Umsatzes kann es vorteilhaft sein, 3-50% Natrium- oder Kaliumacetat zuzugeben, dies ist jedoch nicht unbedingt erforderlich. Gasförmige Alkene der Formel werden eingeleitet, flüssige Alkene entsprechend zudosiert. Die Alkene werden in der Regel in einem Molverhältnis von 1 bis 5 : 1 in Bezug auf das Oxim VI eingesetzt.
   Die Reaktion wird bei Temperaturen von 0 bis 80°C, bevorzugt bei Temperaturen zwischen 20 und 50°C durchgeführt. Die Reaktion wird unter einem Druck von 0 bis 20 bar, bevorzugt bei 0 bis 6 bar durchgeführt.
6. Stufe f) 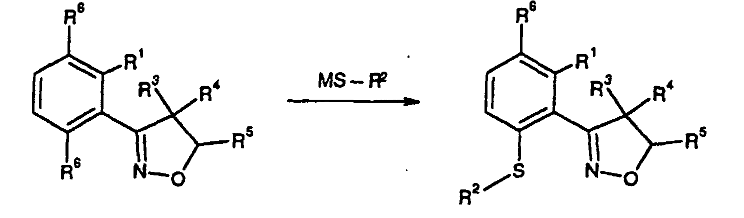
   Die Reaktion von Alkali- bzw. Kupferthioalkylaten mit aromatischen Halogenverbindungen führt zu aromatischen Alkylthioethern.
   Die Umsetzung erfolgt unter den folgenden Bedingungen: Lösungsmittel: Alkohole wie Methanol, Ethanol, Propanol, t.-Butanol, Wasser, Ether wie Dioxan, THF, polar aprotische Lösungsmittel, z.B. N,N-Dialkylformamide, - acetamide, N-Methylpyrrolidon, Dimethylpropylenharnstoff; Tetramethylharnstoff, Acetonitril, Propionitril, Dimethylsulfoxid; bevorzugt: Methanol, DMF, NMP. Temperatur: 0°C bis 170°C, bevorzugt 30°C bis 120°C, bes. bevorzugt 40°C bis 100°C.
   Durchführung: Das Alkalithioalkylat z.B. Natriumthiomethylat kann als Feststoff, als wäßrige bzw. methanolische Lösung eingesetzt werden oder *in situ* aus dem Alkylmercaptan, z. B. Methylmercaptan und einer Base Alkali- oder Erdalkalialkoholat oder -hydroxid, z. B. Natriummethylat, Kaliumethylat, Natriumhydroxid oder Kaliumhydroxid hergestellt und eingesetzt werden. Die Umsetzung kann auch im Vakuum durch zusätzliche Zugabe eines hoch siedenden dipolar aprotichen Lösungsmittels durchgeführt werden unter Abdestillation des niedrigsiedenden Lösungsmittels z.B. Wasser oder Methanol. Durch den Zusatz von Kupferpulver als Katalysator (0,01-10 mol%) läßt sich oftmals eine Vervollständigung und Beschleunigung der Umsetzung erreichen. Die Thioalkylierung wird in der Regel bei 0 bis 100°C, bevorzugt bei 20 - 80°C durchgeführt.
7. Stufe g) 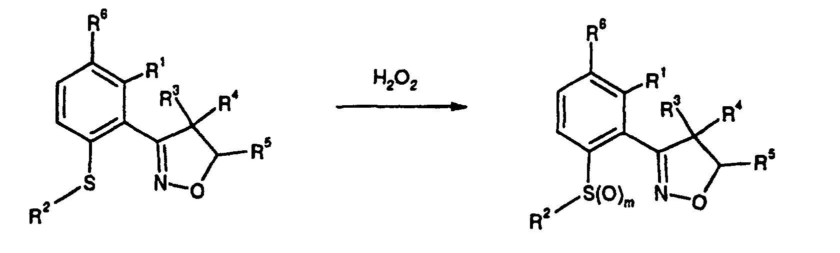
   Die Oxidation erfolgt analog zur Umsetzung des Clorderivats (R¹ = CL), beschrieben in: WO 98/31681 (vgl. S. 8 Zeile 32 bis S. 11, Zeile 25).

In den folgenden Ausführungsbeispielen wird die Erfindung näher erläutert.

### Beispiel 1

### Herstellung 3,6-Dichlor-1,2-xylol

Die Chlorieriung von 1,2-Xylol erfolgt nach literaturbekannten Methoden unter Verwendung von Chlorgas. Als Lösungsmittel für Xylol wird Ethanol verwendet.

### Beispiel 2

### Herstellung von 3,6-Dichlor-2-methylbenzylbromid

170,1 g (0,97 mol) 3,6-Dichlor-1,2-xylol werden in 1180 ml Chlorbenzol vorgelegt und 4,9 g konz. H₂SO₄ und 203,1 g (1,18 mol) 47 % ige Bromwasserstoffsäure zugegeben. Das Gemisch wird auf 70°C geheizt und 0,9 g AIBN zugegeben. Innerhalb von 5 h werden 353,7 g (1,04 mol) 10 % ige Wasserstoffperoxidlösung bei 70-75°C zugefahren, 30 min. bei 70-75°C nachgerührt, zweimal mit 400 ml Wasser, einmal mit 400 ml gesättigter Natriumhydrogencarbonatlösung gewaschen und anschließend das Chlorbenzol abdestilliert.

Man erhält 242,9 g 78,4 %-iges Produkt (9,5 % Edukt, 10,4 % Dibromverbindung). Ausbeute: 77,2 %. GC/MS: m/z: 252.

### Beispiel 3

### Herstellung von 3,6-Dibrom-2-methylbenzylbromid

183,2 g (0,65 mol) 3,6-Dibrom-1,2-xylol werden in 750 ml Chlorbenzol vorgelegt und anschließend 2 g konz. H₂SO₄ und 270,7 g (1,57 mol) 47 % ige Bromwasserstoffsäure zugegeben. Das Reaktionsgemisch wird auf 70 C geheizt und 0,3 g AIBN zugegeben. In 14 h werden 237,7 g (0,7 mol) einer 10 % ige Wasserstoffperoxidlösung bei 75-77 °C zugefahren, 120 min. nachgerührt, 2 x mit 250 ml Wasser, einmal mit 250 ml gesättigter Natriumhydrogencarbonatlösung gewaschen und anschließend das Chlorbenzol abdestilliert.

Man erhält 219,3 g 72,3 % iges Produkt (15,3 % Edukt, 6,4 % Dibromverbindung). Ausbeute: 70,9 %. GC/MS: m/z: 340.

### Beispiel 4

### Herstellung von 3,6-Dichlor-2-methyl-benzaldehyd

122,4 g (0,68 mol) 30 % ige Na-methylatlsg wird in 1030 ml Methanol gelöst und anschließend 56,4 g (0,57 mol) 90 %iges 2-Nitropropan und 188,5 g (0,52 mol) 61,6 % iges 3,6-Dichlor-2-methylbenzylbromid zugegeben. Die Reaktion ist exotherm bis 53°C und wird dann 90 mine nachgerührt. Das Reaktionsgemisch wird auf 2,5 1 Wasser gegeben, mit 10 % iger HCl auf pH 7,0 gestellt, dreimal mit 1 l Essigester extrahiert, organische Phasen vereinigt, zweimal mit 500 ml gesättigte NaCl-Lösung gewaschen, über Na₂SO₄ getrocknet, und im Vakuum eingeengt: Die 161,0 g des Rohprodukts wurden über eine 10 cm Kolonne mit 10 mm Raschigringen destilliert. Die Kristalle aus den letzten Fraktionen wurden filtriert. Man 9,8 g 85,9 % gewünschtes Produkt, 4,5 % und 6,9 % Isomere. Ausbeute:9,7 %. Der Rückstand wurde über eine Spaltrohrkolonne destilliert, es wurden nochmals 3,2 g 94,3%-iges Produkt gewonnen.

### Beispiel 5

### Herstellung von 3,6-Dichlor-2-methyl-benzaldehyd

67,4 g (0,19 mol) 72,2 % iges 3,6-Dichlor-2-methylbenzylbromid wird in 280 ml Acetonitril vorgelegt. Eine Lösung aus 54,0 g (0,46 mol) N-Methylmorpholin-N-oxid und 280 ml Acetonitril wird innerhalb von 25 min bei 0-5°C zugegeben und 1 h bei 0-8°C nachgerührt. Der Niederschlag wird abgesaugt, in 280 ml Acetonitril aufgenommen und anschließend 250 g (0,42 mol) 20 %iges NMO in Acetonitril bei 40°C zugegeben. Das Reaktionsgemisch wird 1 h bei 40 °C nachgerührt, im Vakuum eingeengt, in 250 ml Methylenchlorid aufgenommen, dreimal mit 250 ml Wasser gewaschen, über Na₂SO₄ getrocknet und im Vakuum eingeengt. Man erhält 32,9 g 92,6 %-iges Produkt.

Ausbeute: 84,2 %. ¹H-NMR (CDCl₃): 2,6ppm (s, 3H, Me), 7,2ppm (d, 1H, Arom.-H), 7,45ppm (d, 1H, arom.-H), 10,5ppm (s, 1H, CHO).

### Beispiel 6

### Herstellung von 3,6-Dichlor-2-methyl-benzaldoxim

198 g (0,975 mol) 93%iger 3,6-Dichlor-2-methyl-benzaldehyd und 416,2 g (0,634 mol) 25%ige wäßrige Hydroxylammoniumsulfatlösung werden in 1,5 l Toluol gemischt und auf 80°C aufgeheizt. Anschließend tropft man über 2 h 109,2 g (1,36 mol) NaOH 50%ig so zu, daß der pH-Wert zwischen 3 und 5 liegt. Es wird noch 1 h bei 80°C nachgerührt und anschließend bei 80°C die Phasen getrennt. Die organische Phase wird einmal mit 250 ml Wasser gewaschen. Die organische Phase wird eingeengt und mit Cyclohexan umkristallisiert. Man erhält 165,4 g des Aldoxims (83,3% d. Th.). ¹H-NMR: (DMSO-D₆) : 2,4ppm (s, 3H, Me), 7,4ppm (d, 1H, arom.-H), 7,5ppm (d, 1H, arom.-H), 8,3ppm (s, 1H, NH), 11,7ppm (s, 1H, OH).

### Beispiel 7

### Herstellung von 3,6-Dibrom-2-methyl-benzaldoxim

10 g (0,42 mol) 3,6-Dibrom-2-methyl-benzaldehyd und 178,5 g (0,272 mol) 25%ige wäßrige Hydroxylammoniumsulfatlösung werden in 1,2 l Toluol gemischt und auf 80°C aufgeheizt. Anschließend tropft man über 2 h 109,2 g (1,36 mol) NaOH 50%ig so zu, daß der pH-Wert zwischen 3 und 5 liegt. Es wird noch 1 h bei 80°C nachgerührt, über Nacht bei Raumtemperatur gerührt und anschließend bei 80°C die Phasen getrennt. Die organische Phase wird einmal mit 350 ml Wasser gewaschen. Die organische Phase wird eingeengt und mit Cyclohexan umkristallisiert. Man erhält 113,9 g (93% d. Th.) des Aldoxims.

1H-NMR: (DMSO-D₆): 2,45ppm (s, 3H, Me), 7,5ppm (d, 1H, arom.-H), 7,6ppm (d, 1H, arom.-H), 8,1ppm (s, 1H, NH), 11,65 ppm (s, 1H, OH).

### Beispiel 8

### Herstellung von 3-(3,6-Dichlor-2-methylphenyl)-4,5-dihydroisoxazol

50 g (0,25 mol) 3,6-Dichlor-2-methyl-benzaldoxim werden in einem Druckbehälter in 750 ml Methylenchlorid gelöst. Nach Aufpressen von 16 g Ethylen werden 620 g NaOCl-Lsg. 12,5%ig bei Raumtemperatur zugepumpt und über Nacht Rühren lassen. Nach Entspannen des Druckgefäßes wird die organische Phase abgetrennt und einmal mit Wasser gewaschen, getrocknet und das Lösungsmittel im Vakuum abgezogen. Man erhält 58 g (95%iges Produkt) (95% d. Th.). ¹H-NMR (DMSO-D₆): 2,3ppm (s, 3H, Me), 3,3ppm (t, 2H, CH2), 4,5ppm (t, 2H, CH2), 7,45ppm (d, 1H, arom.-H), 7,6ppm (d, 1H, arom.-H).

### Beispiel 9

### Herstellung von 3-(3,6-Dibrom-2-methylphenyl)-4,5-dihydroisoxazol

68 g (0,23 mol) 3,6-Dibrom-2-methyl-benzaldoxim werden in einem Druckbehälter in 750ml Methylenchlorid gelöst. Nach Aufpressen von 20 g Ethylen werden 620 g NaOCl-Lsg. 12,5%ig bei Raumtemperatur zugepumpt und über Nacht Rühren lassen. Nach Entspannen des Druckgefäßes wird die organische Phase abgetrennt und einmal mit 250 ml Wasser gewaschen, getrocknet und das Lösungsmittel im Vakuum abgezogen. Man erhält 77 g (95%iges Produkt) (99% d. Th.).

### Beispiel 10

### Herstellung von 3-(3-Chlor-2-methyl-6-methylthiophenyl)-4,5-dihydroisoxazol

20 g (0,083 mol) 3-(3,6-Dichlor-2-methylphenyl)-4,5-dihydroisoxazol wird in 120 ml NMP gelöst. Bei 0°C wird über 40 min. 6,7 g (0,09 mol) Natriumthiomethylat zugegeben und anschließend über Nacht nachgerührt. Das Reaktionsgemisch in 360 ml Wasser eingerührt, viermal mit 70 ml Toluol extrahiert, die vereinigten organischen Phasen einmal mit 70 ml Wasser gewaschen und die organische Phase eingeengt. Der Rückstand (ein Isomerengemisch) wurde im Vakuum bei 150-170°C 1 mbar destilliert. Die Hauptfraktion wurde chromatographisch gereinigt. Man erhält 4,5 g eines 83%igen Produktes. (17% d. Th.).

### Beispiel 11

### Herstellung von 3-(3-Brom-2-methyl-6-methylthiophenyl)-4,5-dihydroisoxazol

25 g (0,075 mol) 3-(3,6-Dibrom-2-methylphenyl)-4,5-dihydroisoxazol wird in 12 mol NMP vorgelegt. Bei einer Temperatur von 100°C und einem Vakuum von 100 mbar werden 29,3 g (0,09 mol) 21,5%ige methanolische Natriumthiomethylatlsg. Über 40 min. zugetropft. Das Reaktionsgemisch wird über 3 h bei 100°C nachgerührt. Das Reaktionsgemisch wird in 250 ml Wasser eingerührt und dreimal mit 100 ml Toluol extrahiert. Die vereinigten organischen Phasen werden einmal mit 100 ml Wasser gewaschen und anschließend im Vakuum eingeengt. Man erhält 17,5 g dunkles Öl. Im GC/MS findet man neben anderen Isomeren 52,3% des gewünschten Produktes.
MSm/z: 287.

### Beispiel 12

### Herstellung von 3-(3-Chlor-2-methyl-6-methylsulfonylphenyl)-4,5-dihydroisoxazol

1,4 g (6,2 mmol) 3-(3-Chlor-2-methyl-6-methylthiophenyl)-4,5-dihydroisoxazol werden in 3 ml Eisessig gelöst und 30,7 mg Natriumwolframat-dihydrat zugegeben. Bei 25-40°C werden 2,1 g 18,6 mmol) Wasserstoffperoxid zugetropft und das Reaktionsgemisch 3 h nachgerührt. Anschließend gibt man das Reaktionsgemisch auf 1,5 ml Wasser kühlt auf 0°C ab und saugt den ausgefallenen Niederschlag ab, wäscht fünfmal mit 10 ml Wasser und trocknet im Vakuum. Man erhält 1,23 g des Produktes.

## Patentansprüche

1. Verfahren zur Herstellung von Isoxazolen der Formel I 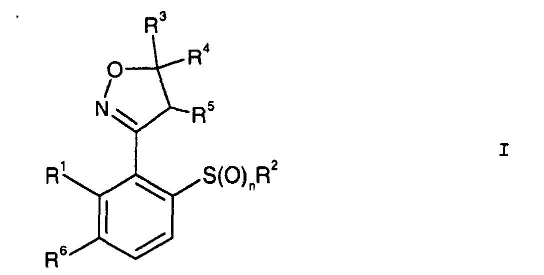 in der die Substituenten folgende Bedeutung haben:
n 0,1 oder 2;
R¹,R² C₁-C₆-Alkyl;
R³,R⁴,R⁵ Wasserstoff, C₁-C₆-Alkyl oder R⁴ und R⁵ bilden zusammen eine Bindung;
R⁶ Cl, Br,
umfassend die aufeinander abfolgende Durchführung der Verfahrensschritte:
a) Halogenierung eines 1,2-Dialkylbenzols der Formel II 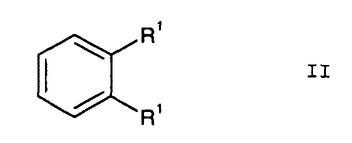 in der die Reste R¹ gleich oder verschieden sein können und die oben angegebene Bedeutung haben, mit Halogenen, insbesondere Chlor, zu den 3,6-Dihalogen-1,2-dialkylbenzolen der Formel III 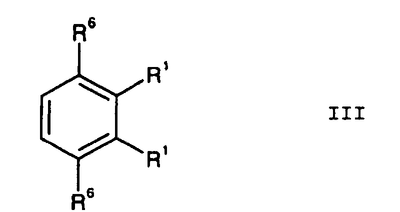
b) Umsetzung eines 3,6-Dihalogen-1,2-dialkylbenzol der Formel III mit Wasserstoffperoxid und einem Halogenierungsmittel, vorzugsweise HBr, zu den Benzylhalogeniden, vorzugsweise Benzylbromiden, der Formel IV 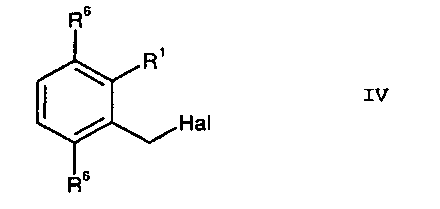 in denen die Reste R¹ und R⁶ die vorgenannte Bedeutung haben; und
c) Oxidation der Benzylhalogenide der Formel IV mit einem Oxidationsmittel zu den Aldehyden der Formel V 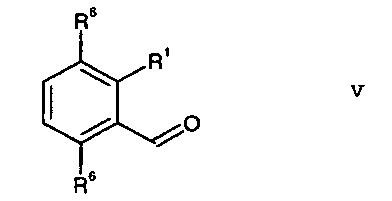 bei denen die Substiuenten R¹ und R⁶ die vorgenannte Bedeutung haben.

2. Verfahren nach Anspruch 1, wobei zusätzlich ein oder mehrere der folgenden Verfahrensschritte
d) Umsetzung der Verbindungen der Formel V mit Hydroxylamin und Base zu den entsprechenden Oximen der Formel VI 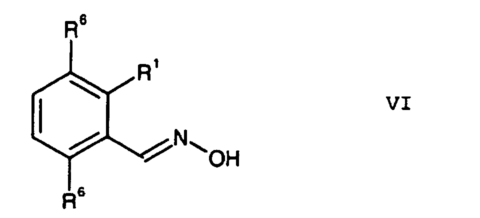 bei denen die Substituenten R¹ und R⁶ die vorgenannte Bedeutung haben;
e) Umsetzung der Oxime der Formel VI mit einem Alken der Formel VII 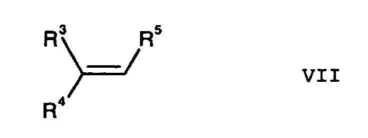 in der R³ bis R⁵ die in Anspruch 1 genannten Bedeutungen haben, vorzugsweise in Gegenwart eines Hypochlorits, zu 4,5-Dihydroisoxazol der Formel VIII 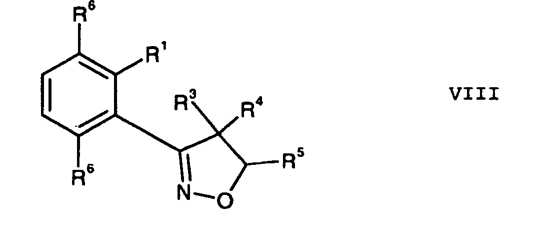 bei denen R¹ und R³ bis R⁶ die in Anspruch 1 genannten Bedeutungen haben;
f) Umsetzung der Verbindung der Formel VIII mit Metallthiolaten der Formel IX
R² ―S⁻ M⁺ IX
in Gegenwart eines geeigneten Lösungsmittels zu den Thioethern der Formel X 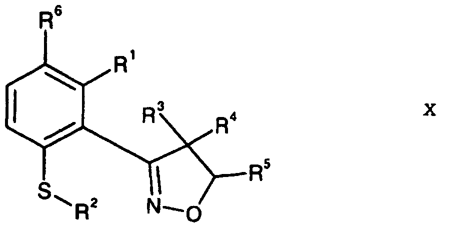 bei denen R¹ bis R⁶ die in Anspruch 1 genannten Bedeutungen haben;
g) Umsetzung der Thioether der Formel X mit einem Oxidationsmittel zu den entsprechenden Alkylsulfonyloder Alkylsulfenylderivaten der Formel I, wobei n die Zahlen 1 oder 2 bedeuten,
durchgeführt werden.

3. Verfahren nach einem der Ansprüche 1 oder 2, wobei R³ Wasserstoff oder Methyl ist.

4. Verfahren nach einem der Ansprüche 1 - 3, wobei R⁴ Wasserstoff oder Methyl ist.

5. Verfahren nach einem der Ansprüche 1 - 4, wobei R⁵ Wasserstoff oder Methyl ist.

6. Verfahren nach einem der Ansprüche 1 - 5, wobei R³, R⁴ und R⁵ Wasserstoff sind.

7. Verfahren nach einem der Ansprüche 1 - 6, wobei R⁶ Brom ist.

8. Verfahren nach einem der Ansprüche 1 - 6, wobei R⁶ Chlor ist.

9. Verfahren nach einem der Ansprüche 1 - 6, wobei einer der Reste R¹ in Formel II eine Methylgruppe bedeutet.

10. Verfahren nach einem der Ansprüche 1 - 6 zur Herstellung einer Verbindung ausgewählt aus:
3-(3-Chlor-2-methyl-6-methylthiophenyl)-4,5-dihydroisoxazol;
3-(3-Brom-2-methyl-6-methylthiophenyl)-4,5-dihydroisoxazol;
3-(3-Chlor-2-methyl-6-methylsulfonylphenyl)-4,5-dihydroisoxazol; oder
3-(3-Brom-2-methyl-6-methylsulfonylphenyl)-4,5-dihydroisoxazol.

11. Verwendung von Verbindungen der Formeln II, III, IV, V, VI, VII, VIII oder IX gemäß den Ansprüchen 1 oder 2 in einem Verfahren zur Herstellung von Verbindungen der Formel I nach den Ansprüchen 1 oder 2 oder deren Vorstufen.

## Claims

1. A process for preparing isoxazoles of the formula I 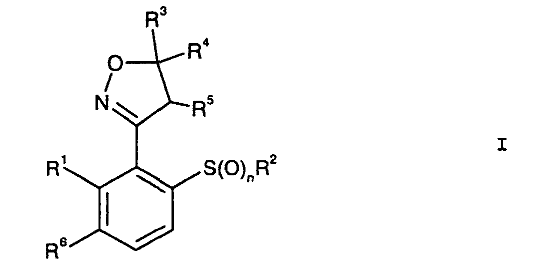 where:
n is 0,1 or 2;
R¹,R² are C₁-C₆-alkyl;
R³,R⁴,R⁵ are hydrogen or C₁-C₆-alkyl, or R⁴ and R⁵ together form a bond;
R⁶ is Cl, Br,
which comprises carrying out the following sequential process steps:
a) halogenation of a 1,2-dialkylbenzene of the formula II 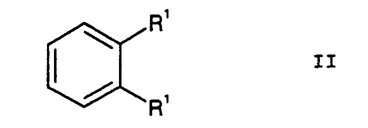 in which the radicals R¹ can be identical or different and are as defined above with halogens, in particular chlorine, to give the 3,6-dihalo-1,2-dialkylbenzenes of the formula III 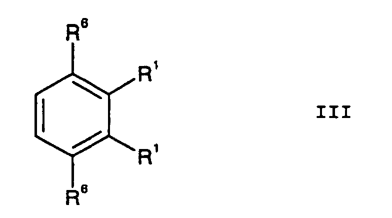
b) reaction of a 3,6-dihalo-1,2-dialkylbenzene of the formula III with hydrogen peroxide and a halogenating agent, preferably HBr, to give the benzyl halides, preferably benzyl bromides, of the formula IV 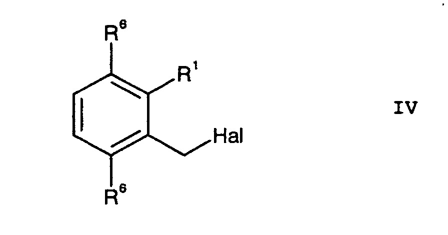 in which the radicals R¹ and R⁶ are as defined above; and
c) oxidation of the benzyl halides of the formula IV with an oxidizing agent to give the aldehydes of the formula V 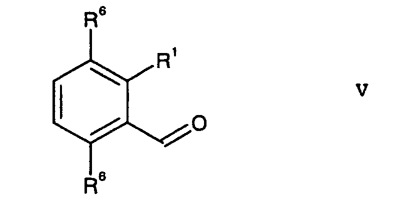 in which the substituents R¹ and R⁶ are as defined above.

2. A process as claimed in claim 1, in which one or more of the following process steps are carried out:
d) reaction of the compounds of the formula V with hydroxylamine and base to give the corresponding oximes of the formula VI 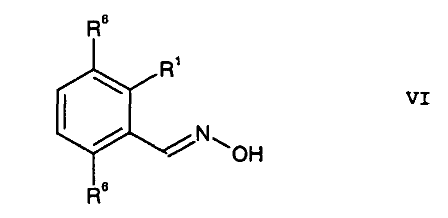 in which the substituents R¹ and R⁶ are as defined above;
e) reaction of the oximes of the formula VI with an alkene of the formula VII 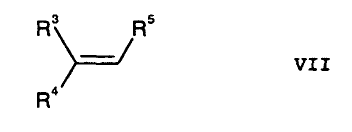 in which R³ to R⁵ are as defined in claim 1, preferably in the presence of a hypochlorite, to give 4,5-dihydroisoxazole of the formula VIII 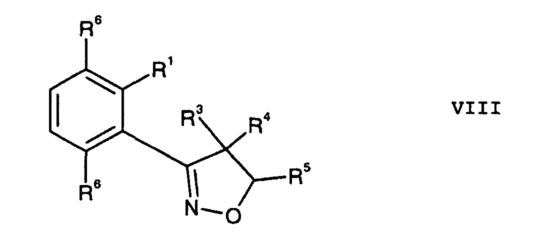 in which R¹ and R³ to R⁶ are as defined in claim 1;
f) reaction of the compound of the formula VIII with metal thiolates of the formula IX
R²― S⁻ M⁺ IX
in the presence of a suitable solvent to give the thioethers of the formula X 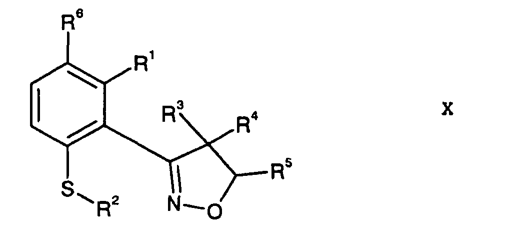 in which R¹ to R⁶ are as defined in claim 1;
g) reaction of the thioethers of the formula X with an oxidizing agent to give the corresponding alkylsulfonyl or alkylsulfenyl derivatives of the formula I where n is the number 1 or 2.

3. A process as claimed in claim 1 or 2 where R³ is hydrogen or methyl.

4. A process as claimed in any of claims 1 - 3 where R⁴ is hydrogen or methyl.

5. A process as claimed in any of claims 1 - 4 where R⁵ is hydrogen or methyl.

6. A process as claimed in any of claims 1 - 5 where R³, R⁴ and R⁵ are hydrogen.

7. A process as claimed in any of claims 1 - 6 where R⁶ is bromine.

8. A process as claimed in any of claims 1 - 6 where R⁶ is chlorine.

9. A process as claimed in any of claims 1 - 6 where one of the radicals R¹ in formula II is a methyl group.

10. A process as claimed in any of claims 1 - 6 for preparing a compound selected from the group consisting of:
3-(3-chloro-2-methyl-6-methylthiophenyl)-4,5-dihydroisoxazole;
3-(3-bromo-2-methyl-6-methylthiophenyl)-4,5-dihydroisoxazole;
3-(3-chloro-2-methyl-6-methylsulfonylphenyl)-4,5-dihydroisoxazole; or
3-(3-bromo-2-methyl-6-methylsulfonylphenyl)-4,5-dihydroisoxazole.

11. The use of compounds of the formulae II, III, IV, V, VI, VII, VIII and IX as claimed in claim 1 or 2 in a process for preparing compounds of the formula I as claimed in claim 1 or 2 or precursors thereof.

## Revendications

1. Procédé de préparation d'isoxazoles de formule I 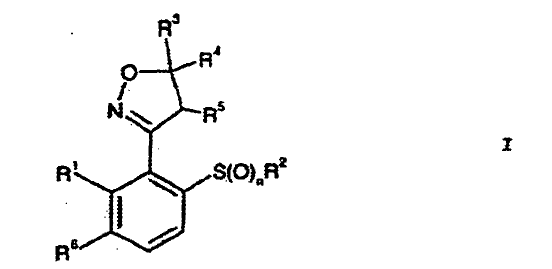 dans laquelle les substituants ont la signification suivante :
n vaut 0, 1 ou 2 ;
R¹, R² alkyle en C₁ à C₆ ;
R³, R⁴, R⁵ hydrogène, alkyle en C₁ à C₆ ou R⁴ et R⁵ forment ensemble une liaison ;
R⁶ Cl, Br ;
comprenant la réalisation successive des étapes opératoires suivantes :
a) halogénation d'un 1,2-dialkylbenzène de formule II 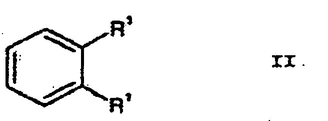 dans laquelle les radicaux R¹ peuvent être identiques ou différents et ont la signification indiquée ci-dessus, avec des halogènes, en particulier du chlore, pour donner les 3,6-dihalogéno-1,2-dialkylbenzènes de formule III 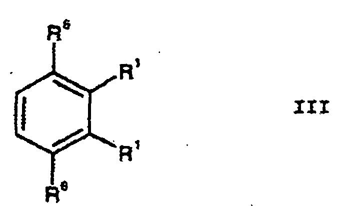
b) réaction d'un 3,6-dihalogéno-1,2-dialkylbenzène de formule III avec du peroxyde d'hydrogène et un agent d'halogénation, de préférence HBr, pour donner les halogénures de benzyle, de préférence les bromures de benzyle, de formule IV 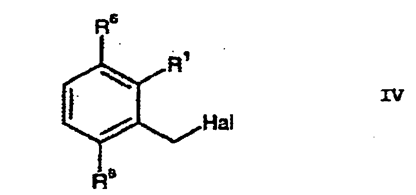 dans laquelle les radicaux R¹ et R⁶ ont la signification mentionnée ci-dessus ; et
c) oxydation des halogénures de benzyle de formule IV avec un oxydant pour donner les aldéhydes de formule V 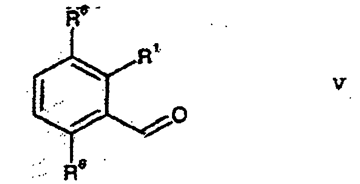 dans laquelle les substituants R¹ et R⁶ ont la signification indiquée ci-dessus.

2. Procédé selon la revendication 1, dans lequel on effectue en outre une ou plusieurs des étapes opératoires suivantes :
d) réaction des composés de formule V avec de l'hydroxylamine et une base pour donner les oximes correspondantes de formule VI 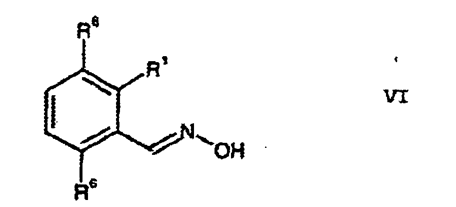 dans laquelle les substituants R¹ et R⁶ ont la signification indiquée ci-dessus ;
e) réaction des oximes de formule VI avec un alcène de formule VII 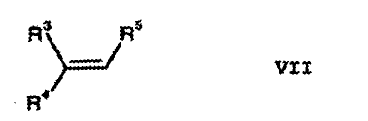 dans laquelle R³ à R⁵ ont les significations données dans la revendication 1, de préférence en présence d'un hypochlorite, pour donner le 4,5-dihydroisoxazole de formule VIII 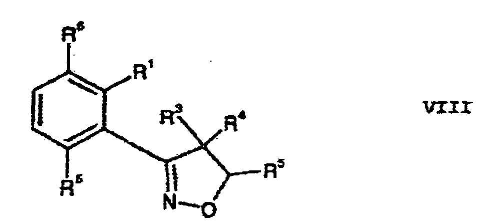 dans laquelle R¹ et R³ à R⁶ ont les significations indiquées dans la revendication 1 ;
f) réaction du composé de formule VIII avec des thiolates métalliques de formule IX
R²―S⁻ M⁺ IX
en présence d'un solvant approprié pour donner les thioéthers de formule X 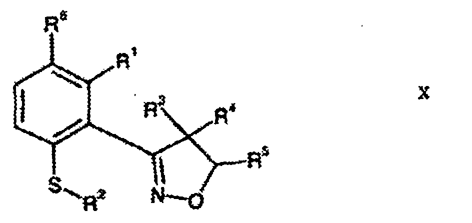 dans laquelle R¹ à R⁶ ont les significations indiquées dans la revendication 1 ;
g) réaction des thioéthers de formule X avec un oxydant pour donner les dérivés alkylsulfonyle ou alkylsulfényle correspondants de formule I, dans laquelle n représente les nombres 1 ou 2.

3. Procédé selon l'une des revendications 1 ou 2, dans lequel R3 est un hydrogène ou un méthyle.

4. Procédé selon l'une des revendications 1 à 3, dans lequel R⁴ est un hydrogène ou un méthyle.

5. Procédé selon l'une des revendications 1 à 4, dans lequel R⁵ est un hydrogène ou un méthyle.

6. Procédé selon l'une des revendications 1 à 5, dans lequel R³, R⁴ et R⁵ représentent un hydrogène.

7. Procédé selon l'une des revendications 1 à 6, dans lequel R⁶ est un brome.

8. Procédé selon l'une des revendications 1 à 6, dans lequel R⁶ est un chlore.

9. Procédé selon l'une des revendications 1 à 6, dans lequel l'un des radicaux R¹ dans la formule II représente un groupe méthyle.

10. Procédé selon l'une des revendications 1 à 6 de préparation d'un composé choisi parmi les suivants :
3-(3-chloro-2-méthyl-6-méthylthiophényl)-4,5-dihydroisoxazole ;
3-(3-bromo-2-méthyl-6-méthylthiophényl)-4,5-dihydroisoxazole ;
3-(3-chloro-2-méthyl-6-méthylsulfonylphényl)-4,5-dihydroisoxazole ; ou
3-(3-bromo-2-méthyl-6-méthylsulfonylphényl)-4,5-dihydroisoxazole.

11. Utilisation de composés de formules II, III, IV, V, VI, VII, VIII ou IX selon les revendications 1 ou 2 dans un procédé de préparation de composés de formule I selon les revendications 1 ou 2 ou de leurs précurseurs.
